# EUROPEAN PATENT SPECIFICATION

(11) **EP 2 398 898 B1**
(45) Date of publication and mention of the grant of the patent: **15.10.2014**
(21) Application number: 10704157.6
(22) Date of filing: 18.02.2010
(51) Int. Cl.: C12N 5/077

(54) **METHOD FOR THE CRYOPRESERVATION OF CELLS, ARTIFICIAL CELL CONSTRUCTS OR THREE-DIMENSIONAL COMPLEX TISSUES ASSEMBLIES**
VERFAHREN ZUR KRYOKONSERVIERUNG VON ZELLEN, KÜNSTLICHE ZELLKONSTRUKTE ODER DREIDIMENSIONALE KOMPLEXE GEWEBEANORDNUNGEN
PROCÉDÉ DE CRYOCONSERVATION DE CELLULES, CONSTRUCTIONS CELLULAIRES ARTIFICIELLES OU ENSEMBLES DE TISSUS COMPLEXES TRIDIMENSIONNELS

(30) Priority: 19.02.2009 EP 09153248
(43) Date of publication of application: 28.12.2011
(73) Proprietor: Naturin GmbH & Co., 69469 Wienheim (DE)
(72) Inventor: SCHMITT, Timo, 70197 Stuttgart (DE); JUST, Lothar, 72379 Hechingen (DE); MASER, Franz, 68159 Mannheim (DE); BECKER, Holger, 64319 Pfungstadt (DE)
(74) Representative: Carpintero Lopez, Francisco
(86) International application number: PCT/EP2010/052068
(87) International publication number: WO 2010/094747

(56) References cited:
- EP-A1- 0 331 786
- WO-A1-2007/059855
- WO-A2-2009/024280
- DATABASE WPI Week 200518 Thomson Scientific, London, GB; AN 2005-173096 XP002526453 & WO 2005/014774 A (NAT INST AGROBIOLOGICAL SCI) 17 February 2005 (2005-02-17)
- MCKAY G C ET AL: "Cryopreservation of rat hepatocyte monolayers: Cell viability and cytochrome P450 content in post-thaw cultures" TOXICOLOGY IN VITRO, vol. 16, no. 1, 1 February 2002 (2002-02-01), pages 71-79, XP002416015 ELSEVIER SCIENCE, GB ISSN: 0887-2333
- ANONYMOUS: "ZRM and Naturin develop innovative biomembranes" BIOTECH/LIFE SCIENCES PORTAL, [Online] 20 February 2008 (2008-02-20), XP002525608 Baden-Wüttemberg Retrieved from the Internet: URL:http://www.mstbw.de/de/region/stern/ma gazin/04521/index.html> [retrieved on 2009-04-27]
- NEIDERT MICHAEL R ET AL: "Cryopreservation of collagen-based tissue equivalents. II. Improved freezing in the presence of cryoprotective agents." TISSUE ENGINEERING, vol. 10, no. 1-2, January 2004 (2004-01), pages 23-32, XP002525607 ISSN: 1076-3279

## Description

### FIELD OF THE INVENTION

The present invention is in the field of cryopreservation of cells and tissue cultures, more specifically it refers to a method for the cryopreservation and long term storage of cells, cell constructs or three-dimensional complex tissue assemblies. The method is based on the use of a collagen cell carrier having a specific composition as well as a very specific thickness and appropriate mechanical properties which are maintained after thawing. The use of such collagen cell carrier (CCC) provides a very suitable support for cryopreservation resulting in very high survival rates after the thawing process and providing cells and tissue assemblies already adhered to a mechanically stable and biocompatible support.

The invention also refers to frozen collagen carrier-cells assemblies and to frozen artificial cell constructs or three-dimensional complex tissue assemblies obtainable by the method of the invention and to the use thereof after thawing.

### BACKGROUND OF THE INVENTION

### Cryopreservation

Cryopreservation plays an important role in the short- and long-term preservation of cells and is thus of central importance in tissue and cell banking. Cryopreserved cells such as human hematopoietic stem cells have been successfully transplanted for the routine clinical treatment of leukemia for decades. Increasingly, cell-based therapies using banked tissues are being employed in other areas of regenerative medicine as well. The great potential of these applications grows as new findings lead to the generation of new products for clinical use.

With the aid of cryotechnology, cell suspensions, adherent cells and even artificially generated tissues can be made available 'just in time' by cell banks. The implementation of such projects, however, requires the availability of the corresponding cell-based regenerated tissues as well as of cryopreservation systems with which adherent cells or complex tissue assemblies can be produced and preserved in their natural three-dimensional form under closely monitored, reproducible and cost-effective conditions.

In order to protect cells during the freezing and thawing processes, special chemicals such as dimethyl sulfoxide (DMSO), trehalose, glycerine or hydroxyethyl starch (HES) are added to the freezing medium.

These so-called cryoprotectants protect the cells and their organelles from membrane damage by ice crystals and from intracellular dehydration during the transition of water from the liquid to the crystalline phase. It is known that freezing and thawing are associated with a loss of vitality and function in the cells, the degree of loss depending on the type of cell being frozen. In conventional procedures, dimethyl sulfoxide is used in concentrations of up to 10% (v/v) either alone or in combination with hydroxyethyl starch. DMSO works primarily in the intracellular compartment by lowering the freezing point, which reduces the formation of ice crystals and thus minimizes cellular dehydration during freezing. In contrast, the action of macromolecules such as hydroxyethyl starch is extracellular by forming a protective envelope for the cells and preventing loss of liquid from the cells during freezing.

### Cell carriers

In addition to cryoprotectants, the right kind of cell carrier is also of main importance in the cryopreservation process. Ideally, its thickness should be approximately that of the cells colonizing it, i.e. less than 150 µm. Because of their greater heat storage capacity, thicker cell carriers can act as a buffer, especially during the freezing and thawing process, producing a temperature gradient which negatively affects standardized freezing or thawing speeds. Reproducibility and standardization become even more important when sensitive and expensive cells, complex cell assemblies or cell-based regenerated tissues must be preserved. Freezing or thawing speed and ambient pressure help determine the extent to which crystallization takes place when the freezing point is reached. In addition, it must be kept in mind that crystallization may also take place inside the material of cell carriers with a certain capacity to absorb fluids. Thinner cell carriers have the additional advantage that cryoprotectants quickly penetrate to the inside.

Several materials have been used as cell carrier for the culture and/or cryopreservation of cells or tissue. Hyaluronic acid, alginate, agarose, fibrin, chitin /chitosan, polylactide (PLA), polyglycolide (PGA) or poly-L-lactic acid (PPLA) are some of the materials known in the prior art as scaffold or carrier for the cultivation and/or cryopreservation of cells.

Collagen-containing carriers have been widely used as matrix for the cultivation of cells as well as for cryopreservation thereof (WO 2007059855 and McKay G.C. et al. Toxicology in vitro, vol. 16(1), 2002:71-79). Collagen is one of the main components in the structure of connective tissues, e.g. skin, blood vessels, ligaments, tendons and cartilage and in the structures of bones and teeth. To date more than 28 different types of collagen have been identified showing only marginal difference between individual species. This fact, together with the fact that the degradation of collagen does not produce any toxic degradation product makes collagen biocompatible and very useful in medicine.

However, the collagen cell carriers used so far for cryopreservation of adherent cells are normally in the form of hydrogels, that is, a jelly-like material with high viscosity and a extremely high water content. Said collagen-based hydrogels have usually been used in different conformations for cryopreservation. The most common and simplest conformation is in monolayer, that is, the cells or tissue are directly seeded in culture dishes coated with a simple collagen gel monolayer. Another widely used conformation for cryopreservation of cells or tissues in collagen based-hydrogels is the sandwich conformation, that is, the cells are cryopreserved between two layers of collagen gel.

Nevertheless, these collagen-based hydrogels have the drawback that they normally have thicknesses over 150 µm which negatively affect cell vitality after thawing for the reasons explained before. In addition, pure collagen hydrogels do not possess sufficient mechanical stability to allow the controlled transfer of the construct into the organism after thawing.

WO2005014774 discloses a collagen hidrogel for culture of cells prepared by fully drying the hydrogel and rehydrolization thereof. The film disclosed in this document has a thickness in the range of µm-1mm, however, the document is completely silent about the suitability of this film for undergoing a cryopreservation process.

Thus, there is a need to develop methods for the cryopreservation of cells, cell constructs or complex tissue assemblies supported by cell carriers having a sufficient mechanical stability after thawing. This would allow, in the case of regenerative medicine, the direct implantation and immobilization of the cells-carrier construct into the damaged organism but conserving at the same time the beneficial effect with regard to biocompatibility and degradability already known for collagen carriers.

All mechanically stable carrier matrices presently available for the cryopreservation of adherent cells are based on synthetic or complex materials or are more than 150 µm thick. An integration of cells in synthetic cell carriers does not take place or is possible only to a limited degree.

The methods for cryopreserving cells, cell constructs and tissue assemblies provided herein, are based on the characteristics of the newly developed, simple and standardized collagen membrane (CCC) having a combination of high mechanical stability and extreme thinness. CCC is a biodegradable cell carrier for cryopreservation that allows the non-destructive freezing of adherent cells as well as the easy transfer of the cell assembly after thawing. The preparation of a CCC as used in the method herein described is taught in the application PCT/EP/2008/006660.

### BRIEF DESCRIPTION OF THE FIGURES

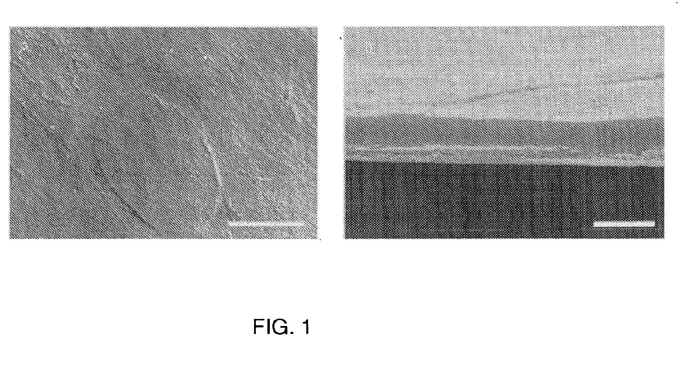

**Figure 1****:** microscopic view of the surface of the CCC. **A:** Electron microscope image of the surface. Scale bar: 500 µm. **B:** Cross section of membrane. Scale bar 10 µm.
**Figure 2****: A:** Force strain of the CCC. continiuous line: dry CCC, dotted line: wet CCC **B**: the tensile test set-up is represented. **C:** geometry of the test specimen.
**Figure 3****: A:** Cryoinsert for the cultivation of adherent cells (lying on its sides) and cryopreservation tubes for storing the insert. **B:** Cryoinsert with collagen-based carrier membrane.
**Figure 4****: A/B:** DAPI nuclear staining of mesenchymal stem cells (MSC) on the top side of the membrane (A) and Caco-2 cells on the bottom (B). Image taken with an inverse fluorescence microscope. Scale bars 100 µm. C: Combined immunocytochemical staining of MSC (Vimentin, red) and Caco-2 cells (Cytokeratin, green). Three dimensional reconstruction with a fluorescence laser scanning microscope. Scale bar: 50 µm.
**Figure 5****: A:** Fetal murine cardiomyocyte cultured on CCC. Alpha-actinin immunocytochemistry after 7 days in culture. **B:** Alpha-actinin immunocytochemistry of cryopreserved cardiomyocytes. Fetal murine cardiomyocytes were cultured on a collagen cell carrier and stored for 3 weeks in liquid nitrogen. Scale bar: 20 µm.
**Figure 6****:** Vimentin and BrdU immunocytochemistry of cryopreserved SAOS-2 cells.The cells were cultured on a collagen cell carrier and stored for 230 days in liquid nitrogen. After thawing, cell were incubated with BrdU and analyzed by immunocytochemistry. The arrows indicate BrdU-positive cells. Scale bar: 40 µm.

### DESCRIPTION OF THE INVENTION

A first object of the present invention refers to a method for the cryopreservation and long term storage of one or several type of cells, cell constructs or three-dimensional complex tissue assemblies comprising:
a) Seeding one or several type of cells or different types of cells conforming the cell constructs or three-dimensional tissue assemblies onto a stable collagen cell carrier (CCC) having a thickness of less than 150 µm, a mass per unit area of 10 to 170 g/m² and the following composition:
   i) collagen [% weight]: 30 to 80,
   ii) amide nitrogen [% weight]: 0.06 to 0.7,
   iii) polyol [% weight]: 0 to 50,
   iv) lipids [% weight]: 0 to 20,
   v) water [% weight]: 5 to 40,
b) culturing the cells until their adherence to the CCC,
c) washing-off non-adherent cells,
d) freezing the collagen cell carrier with adherent cells in a freezing medium.

The method of cryopreservation of the present invention is especially interesting because it allows the freezing of adherent cells or cell constructs or three-dimensional tissue assemblies which after thawing remain attached to the CCC. The CCC-cells assembly or the cell construct or three dimensional tissue construct attached to the mechanically stable support of the collagen film may be thawed whenever needed and provided just in time for their application or use.

In prior art methods, before freezing, the adherent cells were generally detached from the cell culture surface with proteases such as trypsin, washed in a series of centrifugation steps and then frozen after the addition of the cryopreservation medium. When needed the cells were thawed and plated. In other prior art methods, the cells were frozen within or attached to the carrier and after thawing detached from the carrier surface and isolated by the same processes. Nevertheless, both the protease treatment with concomitant destruction of the cell-cell contact and cell-substrate contact and the mechanical stress of centrifuging and pipetting leads to reduced survival rates or damaging of the cells.

The cryopreservation of adherent cells growing on a biocompatible carrier membrane as in the method of the present invention has the great advantage over cell isolation methods that it is no longer necessary to detach the cells from the surface on which they were grown. This increases both the survival rate and the vitality of the cells after thawing. This particularly applies to the cryopreservation of sensitive cells such as primary cardiomyocytes or liver cells. The high survival rate according to the method of this invention has also the advantage that small amounts of cells can be deep-frozen. This is, again, in contrast with cell isolation methods where a minimum number of cells 100.000 is needed to obtain a visible cell pellet after centrifugation, appropriate to be transferred into a fresh culture medium and then seeded.

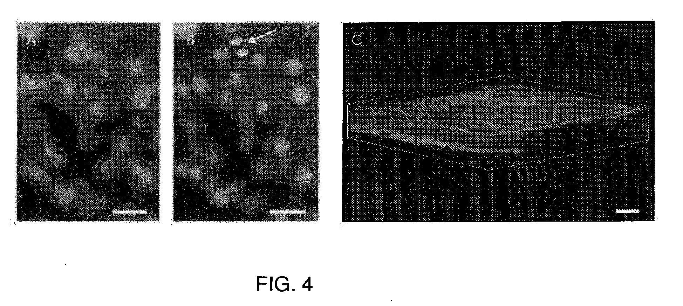

A particular variant or embodiment of the method of the invention comprises the seeding and culturing of one or several types of cells onto each side of the collagen cell carrier in order to obtain a specific spatial conformation thereof at both sides of the collagen film. This variant of the method may be useful when complex tissue conformations will be developed. The collagen interface provides a temporary scaffold for multilayered cellular system of different cells. The controlled layering of cells with individual characteristics and the resulting functionalities provide the foundation for complex in vivo-like cell systems. Figure 4 represents an example of this embodiment where two different type of cells (mesenchymal stem cells and Caco-2 cells) are cultured each onto one of the sides of the CCC.

As expressed before, the advantages of the present method of cryopreservation derive to a great extent from the specific features of the CCC used. The CCC with the above mentioned characteristics provides special chemical and physical properties that make it especially suited not only for the cultivation of cells but also for the cryopreservation thereof. It has been observed that the CCC herein described does neither display any significant loss of biocompatibility nor of mechanical stability after the freezing and thawing processes, even after long-term cryopreservation times.

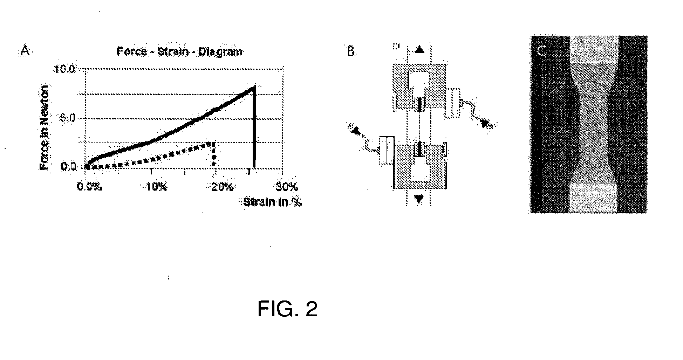

The special mechanical characteristics of the CCC are obtained through air-drying of the collagen composition in the course of the manufacture of the corresponding collagen film. This irreversible process makes it highly stable and resistant. The collagen composition is preferably formed into an air-dried, stable and thin flat film that reaches tensile strengths of around 20 to around 100 N/mm². Figure 2 shows the mechanical behaviour of the CCC in the force-strain-diagram using the tensile test apparatus UTS Software 209.00 V 4.06.04 (Tests according to the German industry standard DIN 53 455). The test setup used to carry out the test is also shown in figure 2.

In particular embodiments where more demanding tensile strengths are needed the collagen composition may be obtained by crosslinking with chemical crosslinking agents such as bifunctional aldehydes like glutaraldehyde, isocyanates like hexamethylene diisocyanate (HMDI), carbodiimides like 1-(3-dimethylaminopropyl)-3-ethylcarbodiimide (EDC) or any other chemical crosslinkers known in the art and not introducing undesired levels of toxicity into the resulting product. Crosslinking may also be achieved by physical methods like dehydrothermal crosslinking or irradiation (UV or ionising radiation), or by enzymatic reactions using, for example, transglutaminase.

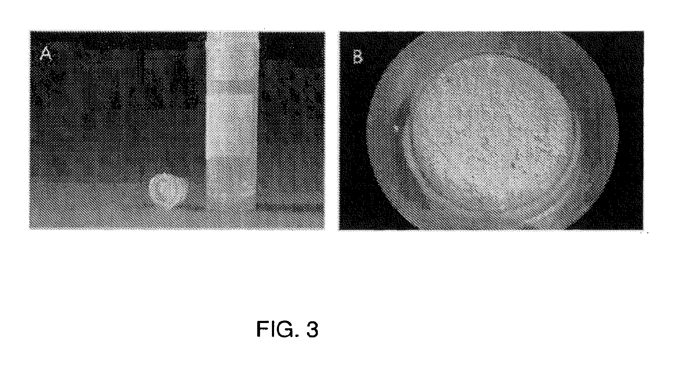

For carrying out the method of the invention the collagen film can be presented in any kind of support for the cultivation of cells such as cell culture plates, dishes or flasks, microbeats. It can also be presented in an insert structure for the wells of a cell culture plate or any other construction type (see figure 3). The latter presentation allows practicing the embodiment of the invention where different types of cells are cultured simultaneously onto each of the sides of the CCC.

In addition to the conformation as a thin flat film, the collagen composition may also be formed as a tubular film for use as cell reservoirs. The collagen tubes can be cryopreserved after been filled with fluid cell suspensions. After thawing, they may be used to transfer regeneration-promoting cell suspensions to a tissue defect in the body.

The thickness of the dry collagen film used in the method of the invention is always less than 150 µm in order not to negatively affect the freezing process. In a preferred embodiment of the invention, the thickness of the dry CCC is less than 80 µm and in a yet preferred embodiment the thickness is equal or less than 40 µm. The low wall thickness allows a quick penetration of the cryoprotectants and also leads to a rapid wash-out thereof after thawing.

Any type of fibrillar collagen from any animal source is useful in the preparation of the CCC used in the method of the invention. Notwithstanding, a preferred embodiment of the invention comprises the use of bovine collagen I obtained from bovine hides or skins. Other particular embodiments comprise a mixture of collagen I and collagen III or a mixture of collagen I and/or III with elastin.

An important component of the collagen composition used in the method of the invention is the polyol. This acts as a humectant which prevents an inappropriate drying-out of the CCC. Although the film is air-dried during its preparation it is convenient that it preserves a certain amount of humidity in order not to become brittle. Many different polyols may be used in the context of the present invention such as glycerol, ethylene glycol, butene diols, propene diols, sorbitol or other hexitols, xylitol or other pentitols and mixtures thereof. A preferred embodiment comprises the use of glycerol or sorbitol or mixtures thereof.

Another component of CCC may be fat. Preferably, the fat applied is essentially of vegetable origin. The use of vegetable oils increases the elasticity of the collagen film.

A particular and preferred embodiment of the invention is represented by a CCC having - in its dry state - a thickness of 20 µm, a mass per unit area of 25 to 35 g/m² and the following composition:
Collagen [% weight]: 50 to 70,
Amide nitrogen [% weight]: 0.14 to 0.4,
Glycerol [% weight]: 12 to 35,
fat [% weight]: 1 to 5,
Sorbitol [% weight] 0 to 20,
Water [% weight]: 5 to 40,

Although it is not an essential feature for carrying out the method of the invention, the CCC may optionally be modified with different type of molecules on its surface. For instance, the CCC may be coated with structural proteins of the extracellular matrix such as laminin, fibronectin, elastin or hyaluronic acid, also with apatite or with functional molecules such as growth factors, cytokines, guidance molecules or antibodies. These factors can influence the cell viability, cell adherence, cell orientation, cell proliferation and cell differentiation.

The specific conditions for culturing the cells to adherence may vary depending on the type of cells. It is substantial to all cells that they are cultivated under submerge conditions in a nutrition media or in a nutrition fog which guarantees that the cells do not dry-out. A cell specific atmosphere and a biocompatible surface like tehone of the CCC used herein, where cells can attach to is additionally needed. Generally a nutrition media, a prokaryote and eukaryote cell specific atmosphere and a specific temperature are needed. The cell culture media and culture conditions on CCC for each cell are the same as described for conventional cell culture applications.

Prior to freezing non-adherent cells are washed-off with one or several washings steps in a buffer solution.

The freezing of the cell-CCC assembly or of the cell construct or three dimensional tissue assemblies in the CCC is carried out in the presence of a cryopreservation medium. This normally comprises a culture medium supplemented with cryprotectants such as dimethyl sulfoxide (DMSO), trehalose glycerine or hydroxyethyl starch (HES). The preferred embodiment of the invention comprises the use of DMSO in the cryopreservation method. The freezing is normally carried out under liquid nitrogen although other known method and/or freezing protocols may be used.

The method of the present invention has been shown to be successful in the cryopreservation of a very broad range of either eukaryotic or prokaryotic cells, whether genetically modified or not.

Among eukaryotic cells several animal, plant or fungi cells have been successfully cultured and deep-frozen. Among prokaryotic cells several bacteria species have also been shown to be viable after thawing.

The method for the cryopreservation of the invention is especially suited for mammal cells, either for primary cells or immortalized cell lines.

The following is a list of the types of cells that could be subjected to the method of cryopreservation of the invention:
a) Stem cells (embryonic, fetal, neonatal, adult) and their progenitor cells
   - Embryonic stem cell lines
   - Induced pluripotent stem cells
   - Mesenchymal stem cells
   - Endothelial stem cell,
   - Hematopoietic stem cells
   - Neural stem cells
   - Neural crest stem cells
   - Gastrointestinal stem cells
b) Somatic cells
   - Endothelial cells,
   - Epithelial cells from stomach and intestine
   - Mammary epithelial cells
   - Melanocytes
   - Lung epithelial cells
   - Renal epithelial cells
   - Keratinocytes
   - Urothelial cells
   - Hepatocytes
   - Corneal cells
   - Lens cells
   - Osteoblasts,
   - Osteocytes
   - Odontoblasts
   - Chondrocytes,
   - Ligament cells
   - Tendon cells
   - Gland cells (pituitary gland cells, salivary gland cells, adrenal gland, exocrine and endocrine pancreatic cells, Leydig cells)
   - Fat cells,
   - Fibroblasts
   - Retinal cells
   - Neurons (Dopaminergic, GABAergic, Glutaminergic, Cholinergic, Adrenergic neurons)
   - Glial cells (Astrocytes, Oligodendrocytes, Schwann cells)
   - Smooth muscle cells
   - Skeletal muscle cells
   - Cardiomyocytes
   - Immune cells (B-lymphocytes, T-lymphocytes, Macrophages, Neutrophils, Dendritic cells, Mast cells, Eosinophils, Basophils, Natural killer cells, M-cells. Microglia)
c) Genetically modified cells
d) Cancer cell lines
   - HeLa, Adenocarcinoma cervix human
   - CCF-STTG1, Astrocytoma brain human
   - Hep G2, Carcinoma, hepatocellular liver human
   - SK-MEL-5, Melanoma, malignant skin human
   - Saos-2, Osteosarcoma bone human
   - WERI-Rb-1, Retinoblastoma eye, retina human
e) Immortalized cell lines
   - NuLi, human bronchial epithelium
   - CuFi, human bronchial epithelium
   - CHON-001, human bone cartilage fibroblast,
   - BJ-5ta, human foreskin fibroblast,
   - hTERT-HME1 (ME16C), human mammary epithelium,
   - hTERT-HPNE, human pancreas duct epithelial-like,
   - hTERT RPE-1, human retinal pigmented epithelium,
   - NeHepLxHT, human liver epithelial-like,
   - T HESCs, human endometrium fibroblast-likehybridoma cell lines
f) Plant cells
   - Brownanthus corallinus
   - Carpanthea pomeridiana
   - Conophytum meyerae
   - Delosperma ecklonis
   - Sesuvium portulacastrum
   - Sphalmanthus trichomotus
   - Amaranthus retroflexus
   - Pleuropetalum darwinii
   - Amaryllidaceae
   - Crinum x powellii hort.
   - Catharanthus longifolius
   - Dictyophleba leonensis
   - Ervatamia coronaria
g) Fungal cells
   Filamentous fungi
      - Dacrymyces deliquescens
      - Fennellomyces linderi
      - Cunninghamella blakesleeana
      - Tolypocladium inflatum
      - Radiomyces spectabilis
      - Wallemia sebi
      - Lophodermium seditiosum
   Yeasts
      - Saccharomyces cerevisiae
      - Candida albicans,
      - Debaromyces japonicus
      - Fellomyces polyborus
      - Brettanomyces abstinens
      - Hyphopichia burtonii
      - Kloeckera brevis
h) Bacteria
   - Acidobacterium capsulatum
   - Escherichia coli
   - Saccharococcus thermophilus
   - Lactobacillus acidophilus
   - Bacillus thuringiensis
   - Pseudomonas aeruginosa
   - Enterococcus faecalis

Another object of the invention is the frozen CCC-cells assembly or frozen artificial cell construct or frozen three-dimensional complex tissue assemblies obtainable by the method of the invention. These represent a potential and practically permanent source of cells, cell constructs or tissue assemblies which may be long-term stored and provided "just in time" when needed. This composite material conserves its three-dimensional arrangement after thawing and guarantees the immobilization of the cells at the site where they are needed.

Finally, it is also an object of the invention the use after thawing of the frozen CCC-cells assembly or frozen artificial cell construct or frozen three-dimensional complex tissue assemblies obtainable by the method of the invention.

A first application comprises the use of the collagen carrier-cells assembly, artificial cell construct or three-dimensional complex tissue assembly after thawing in regenerative medicine, i.e. for in vivo implantation of cells or damaged tissues. In vivo implantation studies have shown that the CCC is biocompatible and does not trigger an immune reaction in the recipient. This, together with mechanical stability and reduced thickness makes these thawed products especially interesting as a cell-colonized implant for diverse clinical applications in animals and humans. Cryopreservation allows to generate the implants ahead of time and to thaw them "just in time" for transplantation.

The high mechanical stability of the CCC (even after thawing) also provides protection and stabilization in the initial phase in the case of transplantations until it is resorbed by the surrounding tissue.

It is preferred that the frozen/thawed implants contain autologous cells instead of heterologous ones in order to avoid or reduce any possible immune reaction of the recipient of the implant.

A second application comprises the use of the collagen carrier-cells assembly, artificial cell construct or three-dimensional complex tissue assembly after thawing for in-vitro test systems. These may be used for applications in basic research or as test systems for pharmacological substances, chemicals or cosmetics and make it possible to analyze cytoactive substances without the use of animal tests. One established, well known system for testing pharmacological substances or cosmetics is the skin model. This model may of course be reproduced and frozen according to the method of the invention.

The possibility of freezing/thawing complex cell assemblies together with their carrier matrices while preserving their three-dimensional structure as in the present invention opens up new options for applications in pharmaceutical or biotechnological industry. Cryopreserved test systems prepared according to the invention may be delivered without any danger of contamination or compromised cell vitality resulting from fluctuating culture conditions during transportation.

The following examples are intended to illustrate the invention but do not have to be considered as a limitation of the scope of the invention.

### Example 1: Preparation of the collagen cell carrier

### 1.1-Production as a flat film according to the invention

50,0 kg of bovine hide splits were mechanically pre-cut and three times washed with water (3 times 50 kg). Subsequently the raw material was submitted to an alkaline treatment in a suspension of 0.29 kg of calcium hydroxide in 50 kg of water at a pH value of 11,8 during150 hours. The alkaline treatment was stopped by the addition of hydrochloric acid (10 wt.-% in water) until the pH of the float reached a value of 0,6. Then the reaction mixture was again rinsed with water until the float adopted a pH of 2,8. The resulting "collagen rinds" were then mechanically processed under temperature control (< 24 °C) into a gel-like viscoelastic mass by grinding them coarsely and pressing the minced material through a series of perforated discs with consecutively smaller diameters of the respective holes. The yield was 78,1 kg of "concentrated" collagen mass.

60,0 kg of this "concentrated" mass was transferred into a vessel equipped with a stirrer and a cooling jacket. Water (376,4 kg) and glycerine (2,15 kg) were added under stirring. At the same time, the pH value was adjusted with hydrochloric acid to 2,9. The mixture was subsequently passed through a homogeniser, deaerated and then extruded through a slit nozzle onto a conveyor belt, on which the resulting gel film passed through a tunnel dryer. Before entering the dryer, it was neutralized using gaseous ammonia, thus raising the pH value of the gel. At the end of the dryer, the dried film was passed through a rehydration zone before it was reeled.

### 1.2- Production as a tubular film according to the invention:

The "concentrated" mass was manufactured analogous to 1.1, but the duration of alkaline treatment was reduced to 48 h. 60,0 kg of the resulting "concentrated" collagen mass were transferred into a kneader and diluted under kneading by the gradual addition of water (36,0 kg) under strict temperature control (< 24 °C). In parallel the pH value of the collagen mass was adjusted to 2,8. The resulting dough was passed over a homogeniser and then stored overnight to relax at 20 °C. The next day, the collagen mass so obtained was extruded through an annular nozzle, thereby producing an endless tubular film. To prevent the tubing from collapsing and to neutralise the collagen, a mixture of air and gaseous ammonia was injected into the tubing at the extrusion head. The inflated tubular film was then transported through a series of washing showers (water) and, in the last sprinkling bath, it was sprinkled with a solution of 4 % of glycerine in water. Finally, the tubular film was passed through a tunnel dryer at the end of which it was laid flat between squeegees and wound up on reels. The procedure described under 1.2 was carried twice using different extrusion heads to yield one tubular film with a diameter of 60 mm and one with a diameter of 115 mm.

A fraction of the tubular films obtained was cut open to yield a flat film.

### 1.3 Adjusting the pH value

The pH value of the flat or tubular films resulting from 1.1 or 1.2 was adjusted off the extrusion lines in the laboratory by using a calcium and magnesium containing phosphate buffer [phosphate buffered saline (PBS) containing Ca⁺⁺ and Mg⁺⁺ (PAA H15-001) to reach the physiological range of pH 7.2 to pH 7.5. To this end, the corresponding collagen film was washed with the buffer system under agitation for up to 5 days. The buffer was exchanged twice a day.

In an alternative procedure, the collagen membranes obtained according to 1.1 or 1.2 were immersed for an hour in a phosphate buffer containing glycerine with a pH value of 7.3 (phosphate buffer: 15.6 g of KH₂PO₄, 71.3 g of Na₂HPO₄ x 2 H₂O and 492.9 g of glycerine were dissolved in 7722 g of distilled water). Afterwards, the processed film was left to drain off and placed into a stentering frame, where it dried overnight at room temperature.

### 1.4 Further optional processing

After a short equilibration in distilled water, the collagen membrane was processed with 100 % acetone to precipitate water-soluble protein fractions. After the removal of the acetone, the dried membrane was washed 3 times for one hour each using the calcium and magnesium-containing phosphate buffer (in g/l: KCI 0.2; KH₂PO₄ 0.2; NaCl 8.0; Na₂HPO₄ anhydrous 1.15; CaCl₂-2H₂O in H15-001 0.132; MgCl₂-2H₂O in H15-001 0.1). To eliminate the buffer salt, the resulting film was again washed 3 times for one hour each in distilled water.

### 1.5 Shaping and sterilisation

The dried collagen flat or tubular films obtained by any of the above-mentioned procedures (1.1 - 1.4) were cut in any way or punched into sheets of any shape or size compatible with the film dimensions. The resulting sheets were then sterilised by means of beta or gamma irradiation at 25 kGy or 50 kGy. Dry tubular films were also cut into cuts and irradiated in the same way.

The following table shows the parameters of some typical films obtained according to examples 1.1 and 1.2 :

| Sample | 1 | 2 | 3 |
|---|---|---|---|
| Manufactured according to | 1.1 | 1.2 | 1.2 |
| Type of film | flat | tubular ø 60 mm | tubular ø 115 mm |
| Collagen (wt.-%) | 67 | 76 | 77 |
| Amide Nitrogen (wt.-% based on dry collagen) | 0,42 | 0,57 | 0,57 |
| Water (wt.-%) | 15 | 12 | 12 |
| Glycerine (wt.-%) | 15 | 10 | 6 |
| Fat (wt.-%) | 2 | 1 | 1 |

| Ash (wt.-%) | 1 | 1 | 2 |
|---|---|---|---|
| pH value | 5,1 | 4,2 | 3,8 |
| Thickness dry film (µm) | 20 | 75 | 115 |
| Weight per unit area (g/m²) | 30 | 115 | 160 |
| Tensile strength longitudinal (N/mm²) | 44 | --* | --* |
| Tensile strength transversal (N/mm²) | 41 | --* | --* |

| | | | |
|---|---|---|---|
| * -- = Not determined | | | |

The following analytical methods were applied:
Quantification of collagen by determination of hydroxiproline / amide nitrogen analogue EP1676595 (Geistlich Söhne AG) / Glycerine over HPLC / fat through Soxhlet extraction / Sorbitol over HPLC / Gravimetric ash after incineration in a muffle furnace for 5 hours at 600°C) / Gravimetric water content after drying in the drying cabinet at 150°C / pH value by snipping the film into small pieces, inserting the snippets in a 5-% NaCl solution and measuring using a glass electrode after 10 minutes / mass per unit area by weighing a 10 cm x 10 cm piece of film with balancing humidity / tensile strength longitudinal (= in machine direction) and transversal by means of a UTS universal testing machine (model 3/205, UTS Testsysteme GmbH) after air-conditioning at 21 °C/ 60% relative humidity of the punched sample body and a traverse speed of 100 mm/min.

### Example 2: Cryopreservation of adherent primary cardiomyocytes

Successful cryopreservation of cultured stem cells and/or primary cardiac cells, e.g. cardiomyocytes is a prerequisite for future cell-based therapies in the field of cardiovascular diseases.

The cryopreservation of primary cardiomyocytes that were adherently cultured on biocompatible scaffolds offers several advantages with respect to cell survival, cellular organization and cell biological function in comparison to frozen single cell suspensions.

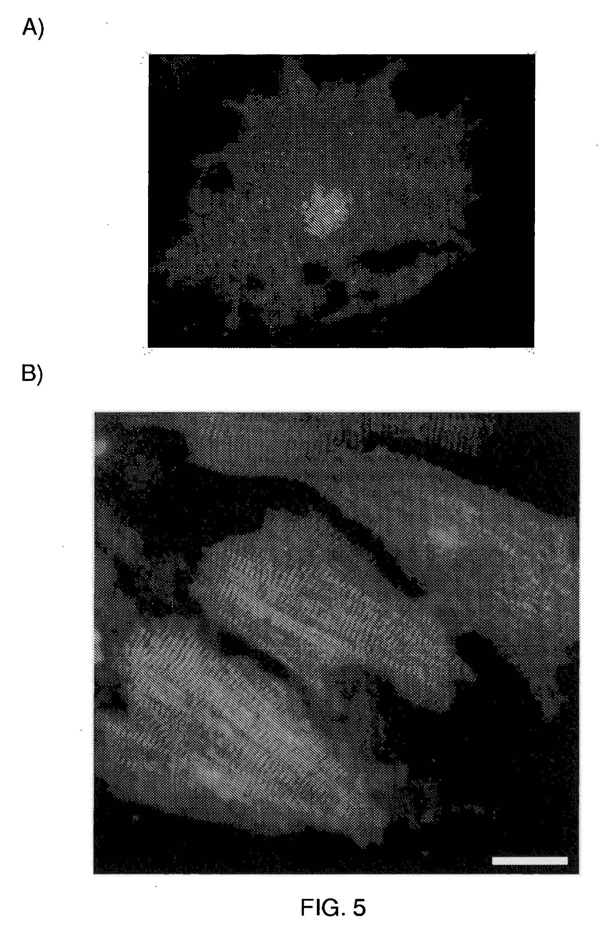

In order to evaluate cryopreservation of adherent primary cardiomyocytes, heart cells were isolated from fetal mouse (E15) and cultured on a collagen cell carrier prepared according to example 1 (see figure 5). Thereafter, cell seeded scaffolds were frozen in liquid nitrogen for 3 weeks and subsequently analyzed after the thawing process.

Fetal murine hearts were prepared and collected in Hanks' balanced salt solution buffer (PAA, Pasching, Austria). Ventricles were dissected and incubated in papain digestion solution (DMEM/F12, PAA) containing 0.05 % (w/v) DNAse I (Sigma, Frickenhausen, Germany) and 0.2 % (w/v) papain (Sigma)) for 30 to 60 min at 37°C. During incubation, tissues were carefully triturated every 15 min with a fire-polished blue tip. To stop enzymatic reaction horse serum was added to a final concentration of 10% (v/v). Afterward, ventricular tissue was triturated with a fire-polished yellow tip to obtain a single cell suspension. This cell suspension was centrifuged at 200 g for 5 min and the remaining cell pellet was resuspended in DMEM/F12 medium supplemented with 10% (v/v) FCS. After a second centrifugation step, cell number and viability were determined in a hemocytometer by Trypan Blue staining. Thereafter, cardiomyocytes were seeded onto the collagen cell carrier prepared in example 1 at a density between 20,000 to 100,000 cells per cm² and cultured for 7 days in a humidified incubator at 37°C and 5% CO₂.The culture medium was renewed every 3 days and consisted of DMEM/F12 (PAA), 10% adult horse serum (Invitrogen, Karlsruhe, Germany), penicillin (100 U/ml, PAA), streptomycin (100 µg/ml, PAA), L-glutamine (2 mM, PAA), insulin/transferrin/selenite mix (1:100, Invitrogen), Albumax (1 mg/ml, Invitrogen), hydrocortisone (1 µM, Sigma), glucagon (14.3 nM, Sigma), 3,3',5'-triiodo-I-thyronine (1 nM, Sigma), ascorbate-2-phosphate (200 µM, Sigma), linoleic acid (20 µM, Sigma), estradiol (10 nM, Sigma). Under these culture conditions adherent cardiomycytes demonstrated their autonomous beating capacity.

For cryopreservation, cell-seeded scaffolds were transferred into cryotubes (Nalge Nunc International Corp., Roskilde, Denmark) and culture medium was replaced by cryomedium (HEPES buffered DMEM supplemented with Penicillin/Streptomycin, 20% (v/v) FCS and 10% (v/v) DMSO (Sigma)). Scaffolds containing cryotubes were cooled using a standardized freezing container ("Mr. Frosty", Nalge Nunc International Corp., Roskilde, Denmark). Cooling, storage and thawing process were performed according to manufacturer's recommendations.

Cell-seeded scaffolds were stored for 22 days in liquid nitrogen below -150°C. Thereafter, constructs were thawed and washed 3 times in pre-warmed culture medium. Within the first two days after thawing, cultured cardiomyocytes started their autonomous cell contractions. The contractility of thawed cell-seeded collagen cell carriers (26 contractions per minute) was documented by video microscopy.

This is the first reference in the art where cardiomyocytes seeded on a collagen cell carrier restart their autonomous cell contraction after having undergone cryopreservation and subsequent thawing.

### Example 3: Long-term cryopreservation of adherent SAOS-2 cells

To analyze long-term cryopreservation of adherent cells, the osteosarcoma cell line SAOS-2 was cultured on a collagen cell carrier prepared according to example 1 and stored for 230 days in liquid nitrogen. After the thawing process, adherent cells were analyzed by a cell proliferation assay.

SAOS-2 cells were seeded onto the collagen cell carrier of example 1 at a density of 25,000 per cm² and cultured for 3 days in a humidified incubator at 37°C and 5% CO₂. The culture medium consisted of HEPES buffered DMEM (PAA) supplemented with Penicillin/Streptomycin and 10% (v/v) FCS. For cryopreservation, these cell-seeded scaffolds were transferred into cryotubes and culture medium was replaced by cryomedium (HEPES buffered DMEM supplemented with Penicillin/Streptomycin, 20% (v/v) FCS and 10% (v/v) DMSO (Sigma)). Collagen cell carrier containing cryotubes were cooled using a standardized freezing container ("Mr. Frosty", Nalge Nunc International Corp.). Cooling, storage and thawing process were performed according to manufacturer's recommendations.

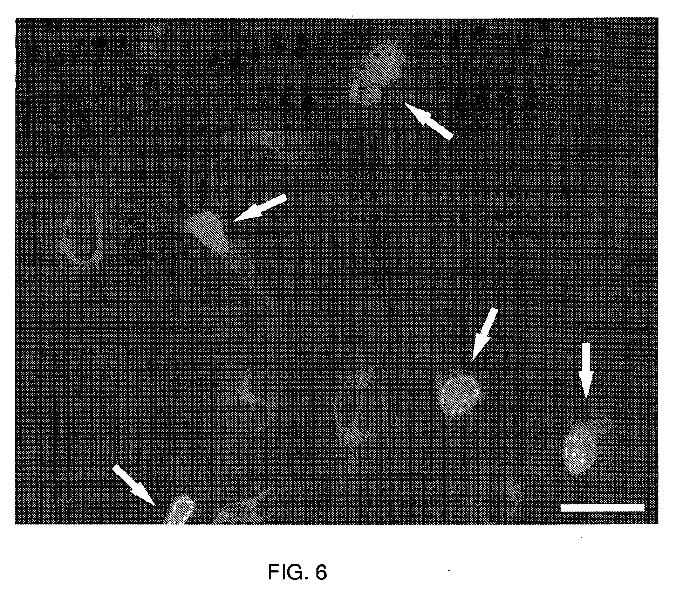

Cell-seeded scaffolds were stored in liquid nitrogen below -150°C for 230 days. Thereafter, constructs were thawed, washed 3-times in pre-warmed culture medium, and kept for additional 4 days in culture. To analyze the proliferation capacity of thawed cells, bromodeoxyuridine (BrdU) proliferation assay (Roche, Mannheim, Germany) was performed according to manufacturer's recommendations. Thus, adherent SAOS-2 cells were incubated for one hour with BrdU (10 µM), fixed with ice-cold 70 % (v/v) ethanol and stained by BrdU-immunofluorescence. Quantification of BrdU-positive cells revealed that 41% ± 7 of total cell population incorporated BrdU during the S-Phase of the cell cycle within 1 hour of culturing (see figure 6).

The data demonstrate that adherent cells seeded on collagen cell carrier maintain their proliferation capacity on a high level even after long-term cryopreservation.

### Example 4: Comparison of the performance of various collagen matrices in cell culture of Saos-2 cells

Rothamel et.al, (2003 Clin. Oral Imbl. Res. 15:443-449) reported on the different performances of commercially available collagen membranes in cell culture experiments. More specifically, they seeded Saos-2 cells (200/mm²) on four different collagen membranes and counted the number of cells present on the membrane after 7 days of cultivation. The results obtained are summarized as follows:

| | Cell count/mm² at the end of the trial * | % of control |
|---|---|---|
| | | |
| Control (CD) | 453 | 100 |
| BioGide (BG) | 94 | 21 |
| BioMend (BM) | 0 | |
| Ossix (OS) | 41 | 9 |
| TutoDent (TD) | 84 | 19 |

| | | |
|---|---|---|
| * for methodological details see original literature | | |

Compared to cultivation on cell culture-treated plastic as well as to the initial number of cells seeded, the cell count on the collagen membranes investigated was severely lower (0% - 21 %). Moreover, the shape of the cells was reported to be roundish indicating low adherence efficiency to the matrix.

To assess the performance of yet another commercially available collagenic cell carrier, "Collagen vitrigel" (Asahi Glass Co., LTD., Tokyo, Japan), Saos-2 cells were seeded (250/mm²) and cultured for 6 days following the instructions of the product leaflet (n=4):

| | Cell count/mm² at the end of the trial * | % of control |
|---|---|---|
| | | |
| Control (CD) | 1269 | 100 |
| Collagen Vitrigel | 579 | 46 |

Cultivation on "Collagen vitrigel" also resulted in a cell number significantly lower (46 %) compared to cell culture-treated plastic. In conjunction with the observation that the cells also revealed a round morphology this matrix appears to have limited abilities for the attachment and expansion of adherent cells.

In an analogous experiment Saos-2 cells were seeded (250/mm²) on the Collagen Cell Carrier (CCC) forming part of the cryopreservation method of the present invention. After 4 days of culture the following data were obtained (n = 6):

| | Cell count/mm² at the end of the trial * | % of control |
|---|---|---|
| | | |
| Control (CD) | 369 | 100 |
| Collagen Cell Carrier (CCC) | 315 | 85 |

After 4 days of culture the cell number on the CCC was just slightly lower (85%) compared to cell culture-treated plastic. Under the microscope the cells showed a flat, extended shape corroborating the findings that the CCC matrix offers excellent environmental conditions to promote attachment and proliferation of adherence-dependent cells.

Overall, the results reveal that there are tremendous differences in the performance of different collagen matrices in cell culture. The use of a Collagen Cell Carrier (CCC) with the specific features addressed in example 1 represents a significant improvement over cell culturing on other collagen matrices. Its use as a matrix in cryopreservation, therefore, represents a significant methodological improvement as compared to the prior art.

### Example 5: Use of "Collagen vitrigel" (Asahi Glass Co., LTD., Tokyo, Japan) in a cryopreservation experiment

Saos-2 cells were seeded at a density of 25.000/cm² onto a collagen matrix known as "Collagen vitrigel" (Asahi Glass Co., LTD., Tokyo, Japan) and cultured for 4 days in a humidified incubator at 37 °C and 5 % CO₂. The culture medium consisted of HEPES buffered DMEM (PAA) supplemented with 1 % Penicillin/Streptomycin, 1 % L Glutamine and 10 % (v/v) FCS. For cryopreservation the cell seeded scaffolds were cut out of their plastic support ring and transferred into cryotubes. Further proceeding was analogous to that disclosed in example 3. Cell-seeded scaffolds were stored in liquid nitrogen below -150 °C for 8 days. Thereafter, constructs were thawed, washed 3-times in pre-warmed culture medium and kept for an additional 3 days in culture. 984.000 dead cells and 112.600 living cells were found in the supernatant after washing. Immunohistological staining with BrdU and DAPI revealed that there were no cells adhering to the cell carrier any longer.

By comparing the results of examples 2 and 3 with those obtained in the present example 5, the superiority of a cryopreservation method including specifically the Collagen Cell Carrier as herein described becomes evident.

## Claims

1. Method for the cryopreservation and long term storage of one or several types of cells, cell constructs or three-dimensional complex tissues assemblies comprising:
a) Seeding one or several types of cells or the different types of cells conforming the cell constructs or three-dimensional tissue assemblies onto a stable collagen cell carrier having a thickness of less than 150 µm, a mass per unit area of 10 to 170 g/m² and the following composition:
i) collagen [% weight]: 30 to 80,
ii) amide nitrogen [% weight]: 0.06 to 0.7,
iii) polyol [% weight]: 0 to 50,
iv) lipids [% weight]: 0 to 20,
v) water [% weight]: 5 to 40,
b) culturing the cells until their adherence to the collagen cell carrier,
c) washing-off non adherent cells,
d) freezing the collagen cell carrier with adherent cells in a freezing medium.

2. Method according to claim 1 where, one type or several types of cells forming a desired cell construct or three dimensional tissue assembly are seeded onto both sides of the collagen cell carrier.

3. Method according to claim 1 or 2 where the collagen of the collagen cell carrier is crosslinked.

4. Method according to claim 1 or 2 where the thickness of the collagen cell carrier is less than 80 µm or less than 40 µm.

5. Method according to claim 1 or 2 where the collagen is type I collagen or a mixture of collagen I and collagen III or a mixture of collagen I and/or III with elastin.

6. Method according to claim 1 or 2 where the polyols are selected from glycerol, ethylene glycol, butene diols, propene diols, sorbitol or other hexitols, xylitol or other pentitols and mixtures thereof.

7. Method according to claim 1 or 2 where the lipids are vegetable oils.

8. Method according to claim 1 or 2 where the collagen cell carrier may be coated with structural proteins of the extracellular matrix, preferably laminins, fibronectin, elastin or hyaluronic acid or with apatite or with functional molecules selected from growth factors, cytokines, guidance molecules or antibodies.

9. Method according to claim 1 or 2 where the freezing medium comprises dimethyl sulfoxide (DMSO), trehalose, glycerine or hydroxyethyl starch (HES).

10. Method according to claim 1 or 2 where the adherent cells are eukaryotic or prokaryotic cells or genetically modified eukaryotic or prokaryotic cells.

11. Method according to claim 10 where the eukaryotic cells are plant, animal or fungal cells.

12. Method according to claim 11 where the animal cells are mammalian cells, either primary cells or immortalized cells, preferably human cells.

13. Method according to any of claims 10 to 12 where the cells are selected from stem cells such as non-human embryonic stem cell lines, induced pluripotent stem cells, mesenchymal stem cells, endothelial stem cell, hematopoietic stem cells neural stem cells, neural crest stem cells or gastrointestinal stem cells; somatic cells such as endothelial cells, epithelial cells from stomach and intestine, mammary epithelial cells, melanocytes, lung epithelial cells, renal epithelial cells, keratinocytes, urothelial cells, hepatocytes, corneal lens cells, osteoblasts, osteocytes, odontoblasts, chondrocytes, ligament cells, tendon cells, pituitary gland cells, salivary gland cells, adrenal gland cells, exocrine and endocrine pancreatic cells, Leydig cells, fat cells, fibroblasts, retinal cells, dopaminergic neurons, GABAergic neurons, glutaminergic neurons, cholinergic neurons, adrenergic neurons, astrocytes, oligodendrocytes, Schwann cells, smooth muscle cells, skeletal muscle cells, cardiomyocytes, B-lymphocytes, T-lymphocytes, macrophages, neutrophils, dendritic cells, mast cells, eosinophils, basophils, natural killer cells, M-cells or microglia; genetically modified cells; cancer cell lines such as HeLa, CCF-STTG1, Hep G2, SK-MEL-5, Saos-2 or WERI-Rb-1; immortalized cell lines such as NuLi, CuFi, CHON-001, BJ-5ta, hTERT-HME1 (ME16C), hTERT-HPNE, hTERT RPE-1, NeHepLxHT or HESCs; plant cells such as cells from Brownanthus corallinus, Carpanthea pomeridiana, Conophytum meyerae, Delosperma ecklonis, Sesuvium portulacastrum, Sphalmanthus trichomotus, Amaranthus retroflexus, Pleuropetalum darwinii, Amaryllidaceae, Crinum x powellii hort., Catharanthus longifolius, Dictyophleba leonensis or Ervatamia coronaria; fungal cells such as Dacrymyces deliquescens, Fennellomyces linderi, Cunninghamella blakesleeana, Tolypocladium inflatum, Radiomyces spectabilis, Wallemia sebi, Lophodermium seditiosum, Saccharomyces cerevisiae, Candida albicans, Debaromyces japonicus, Fellomyces polyborus, Brettanomyces abstinens, Hyphopichia burtonii or Kloeckera brevis; bacteria cells such as Acidobacterium capsulatum, Escherichia coli, Saccharococcus thermophilus, Lactobacillus acidophilus, Bacillus thuringiensis, Pseudomonas aeruginosa or Enterococcus faecalis.

14. Frozen collagen cell carrier-cells assembly or frozen artificial cell construct or three-dimensional complex tissue assembly obtainable by the method of claim 1 or 2, wherein the frozen collagen cell carrier cells assembly or frozen artificial cell construct or three dimensional complex tissue assembly present the same three dimensional structure after thawing.

15. Thawed collagen carrier-cells assembly, artificial cell construct or three-dimensional complex tissue assembly according to claim 14 for use in regenerative medicine.

16. Use of thawed collagen carrier-cells assembly, thawed artificial cell construct or three-dimensional complex tissue assembly according to claim 14 for basic research or for in-vitro test systems such as test systems for pharmacological substances, chemicals or cosmetics.

## Patentansprüche

1. Verfahren zur Kryokonservierung und Langzeitlagerung von einer oder mehreren Arten von Zellen, Zellkonstrukten oder dreidimensionalen komplexen Gewebeverbänden, umfassend:
a) Animpfen einer oder mehrerer Arten von Zellen oder der unterschiedlichen Arten von Zellen, die den Zellkonstrukten oder dreidimensionalen Gewebeverbänden entsprechen, auf einen stabilen Kollagen-Zellträger mit einer Dicke von weniger als 150 µm, einer Masse pro Einheitsfläche von 10 bis 170 g/m² und der folgenden Zusammensetzung:
(i) Kollagen [Gewichts-%]: 30 bis 80,
(ii) Amidstickstoff [Gewichts-%]: 0,06 bis 0,7,
(iii) Polyol [Gewichts-%]: 0 bis 50,
(iv)Lipide [Gewichts-%]: 0 bis 20,
(v) Wasser [Gewichts-%]: 5 bis 40,
b) Züchten der Zellen bis zu ihrer Adhäsion an dem Kollagen-Zellträger,
c) Abwaschen der nicht adhärenten Zellen,
d) Einfrieren des Kollagen-Zellträgers mit adhärenten Zellen in einem Einfriermedium.

2. Verfahren gemäß Anspruch 1, wobei eine Art oder mehrere Arten von Zellen, die ein gewünschtes Zellkonstrukt oder einen gewünschten dreidimensionalen Gewebeverband bilden, auf beide Seiten des Kollagen-Zellträgers angeimpft werden.

3. Verfahren gemäß Anspruch 1 oder 2, wobei das Kollagen des Kollagen-Zellträgers vernetzt ist.

4. Verfahren gemäß Anspruch 1 oder 2, wobei die Dicke des Kollagen-Zellträgers weniger als 80 µm oder weniger als 40 µm beträgt.

5. Verfahren gemäß Anspruch 1 oder 2, wobei es sich bei dem Kollagen um Kollagen Typ I oder ein Gemisch von Kollagen I und Kollagen III oder ein Gemisch von Kollagen I und/oder III mit Elastin handelt.

6. Verfahren gemäß Anspruch 1 oder 2, wobei die Polyole ausgewählt sind aus Glycerin, Ethylenglycol, Butendiolen, Propendiolen, Sorbitol oder anderen Hexitolen, Xylitol oder anderen Pentitolen und Gemischen davon.

7. Verfahren gemäß Anspruch 1 oder 2, wobei es sich bei den Lipiden um Pflanzenöle handelt.

8. Verfahren gemäß Anspruch 1 oder 2, wobei der Kollagen-Zellträger mit Strukturproteinen der extrazellulären Matrix, vorzugsweise Lamininen, Fibronektin, Elastin oder Hyaluronsäure oder mit Apatit oder mit funktionellen Molekülen, ausgewählt aus Wachstumsfaktoren, Zytokinen, Leitmolekülen oder Antikörpern, beschichtet sein kann.

9. Verfahren gemäß Anspruch 1 oder 2, wobei das Einfriermedium Dimethylsulfoxid (DMSO), Trehalose, Glycerin oder Hydroxyethylstärke (HES) umfasst.

10. Verfahren gemäß Anspruch 1 oder 2, wobei es sich bei den adhärenten Zellen um eukaryotische oder prokaryotische Zellen oder genetisch modifizierte eukaryotische oder prokaryotische Zellen handelt.

11. Verfahren gemäß Anspruch 10, wobei es sich bei den eukaryotischen Zellen um Pflanzen-, Tier- oder Pilzzellen handelt.

12. Verfahren gemäß Anspruch 11, wobei es sich bei den Tierzellen um Säugerzellen, entweder primäre Zellen oder immortalisierte Zellen, vorzugsweise menschliche Zellen, handelt.

13. Verfahren gemäß einem der Ansprüche 10 bis 12, wobei die Zellen ausgewählt sind aus Stammzellen, wie zum Beispiel nicht menschlichen embryonalen Stammzelllinien, induzierten pluripotenten Stammzellen, mesenchymalen Stammzellen, endothelialen Stammzellen, hämatopoetischen Stammzellen, neuralen Stammzellen, Neuralleisten-Stammzellen oder gastrointestinalen Stammzellen; somatischen Zellen, wie zum Beispiel Endothelzellen, Epithelzellen aus Magen und Darm, Milchdrüsenepithelzellen, Melanozyten, Lungenepithelzellen, Nierenepithelzellen, Keratinozyten, Urothelzellen, Hepatozyten, cornealen Linsenzellen, Osteoblasten, Osteozyten, Odontoblasten, Chondrozyten, Bänderzellen, Sehnenzellen, Hypophysenzellen, Speicheldrüsenzellen, Nebennierenzellen, exokrinen und endokrinen Pankreaszellen, Leydig-Zellen, Fettzellen, Fibroblasten, Retinazellen, dopaminergen Neuronen, GABAergen Neuronen, glutaminergen Neuronen, cholinergen Neuronen, adrenergen Neuronen, Astrozyten, Oligodendrozyten, Schwann-Zellen, glatten Muskelzellen, Skelettmuskelzellen, Kardiomyozyten, B-Lymphozyten, T-Lymphozyten, Makrophagen, Neutrophilen, dendritischen Zellen, Mastzellen, Eosinophilen, Basophilen, natürlichen Killerzellen, M-Zellen oder Mikroglia; genetisch modifizierten Zellen; Krebszelllinien, wie zum Beispiel HeLa, CCF-STTG1, Hep G2, SK-MEL-5, Saos-2 oder WERI-Rb-1; immortalisierten Zelllinien, wie zum Beispiel NuLi, CuFi, CHON-001, BJ-5ta, hTERT-HME1 (ME16C), hTERT-HPNE, hTERT RPE-1, NeHepLxHT oder HESCs; Pflanzenzellen, wie zum Beispiel Zellen von Brownanthus corallinus, Carpanthea pomeridiana, Conophytum meyerae, Delosperma ecklonis, Sesuvium portulacastrum, Sphalmanthus trichomotus, Amaranthus retroflexus, Pleuropetalum darwinii, Amaryllidaceae, Crinum x powellii hort., Catharanthus longifolius, Dictyophleba leonensis oder Ervatamia coronaria; Pilzzellen, wie zum Beispiel Dacrymyces deliquescens, Fennellomyces linderi, Cunninghamella blakesleeana, Tolypocladium inflatum, Radiomyces spectabilis, Wallemia sebi, Lophodermium seditiosum, Saccharomyces cerevisiae, Candida albicans, Debaromyces japonicus, Fellomyces polyborus, Brettanomyces abstinens, Hyphopichia burtonii oder Kloeckera brevis; Bakterienzellen, wie zum Beispiel Acidobacterium capsulatum, Escherichia coli, Saccharococcus thermophilus, Lactobacillus acidophilus, Bacillus thuringiensis, Pseudomonas aeruginosa oder Enterococcus faecalis.

14. Gefrorener Kollagen-Zellträger-Zellen-Verband oder gefrorenes künstliches Zellkonstrukt oder dreidimensionaler komplexer Gewebeverband, zu erhalten durch das Verfahren nach Anspruch 1 oder 2, wobei der gefrorene Kollagen-Zellträger-Zellen-Verband oder das gefrorene künstliche Zellkonstrukt oder der dreidimensionale komplexe Gewebeverband nach dem Auftauen dieselbe dreidimensionale Struktur darstellen.

15. Aufgetauter Kollagenträger-Zellen-Verband, künstliches Zellkonstrukt oder dreidimensionaler komplexer Gewebeverband gemäß Anspruch 14 zur Verwendung bei der regenerativen Medizin.

16. Verwendung eines aufgetauten Kollagenträger-Zellen-Verbands, eines aufgetauten künstlichen Zellkonstrukts oder dreidimensionalen komplexen Gewebeverbands gemäß Anspruch 14 für die Grundlagenforschung oder für in-vitro-Testsysteme, wie zum Beispiel Testsysteme für pharmakologische Substanzen, Chemikalien oder Kosmetika.

## Revendications

1. Procédé de cryoconservation et de stockage à long terme d'un ou plusieurs types de cellules, constructions de cellules ou assemblages de tissus complexes tridimensionnels consistant à :
a) ensemencer un ou plusieurs types de cellules ou les différents types de cellules formant des constructions cellulaires ou des assemblages de tissus tridimensionnels sur un support cellulaire de collagène stable ayant une épaisseur inférieure à 150 µm, une masse par surface unitaire de 10 à 170 g/m² et la composition suivante :
i) collagène [% en poids] : 30 à 80,
ii) azote amidé [% en poids] : 0,06 à 0,7,
iii) polyol [% en poids] : 0 à 50,
iv) lipides [% en poids] : 0 à 20,
v) eau [% en poids] : 5 à 40,
b) cultiver les cellules jusqu'à leur adhérence au support cellulaire de collagène,
c) éliminer par lavage les cellules non adhérentes,
d) congeler le support cellulaire de collagène avec les cellules adhérentes dans un milieu de congélation.

2. Procédé selon la revendication 1, dans lequel un type ou plusieurs types de cellules formant une construction de cellules ou un assemblage de tissus tridimensionnel souhaité(e) sont ensemencés sur les deux côtés du support cellulaire de collagène.

3. Procédé selon la revendication 1 ou 2, dans lequel le collagène du support cellulaire de collagène est réticulé.

4. Procédé selon la revendication 1 ou 2, dans lequel l'épaisseur du support cellulaire de collagène est inférieure à 80 µm ou inférieure à 40 µm.

5. Procédé selon la revendication 1 ou 2, dans lequel le collagène est un collagène de type I ou un mélange de collagène de type I et de collagène de type III ou un mélange de collagène de type I et/ou de type III avec de l'élastine.

6. Procédé selon la revendication 1 ou 2, dans lequel les polyols sont choisis parmi le glycérol, l'éthylène glycol, les butène diols, les propène diols, le sorbitol ou d'autres hexitols, le xylitol ou d'autres pentilols et des mélanges de ceux-ci.

7. Procédé selon la revendication 1 ou 2, dans lequel les lipides sont des huiles végétales.

8. Procédé selon la revendication 1 ou 2, dans lequel le support cellulaire de collagène peut être revêtu de protéines structurelles de la matrice extracellulaire, de préférence les laminines, la fibronectine, l'élastine ou l'acide hyaluronique ou avec de l'apatite ou avec des molécules fonctionnelles choisies parmi les facteurs de croissance, les cytokines, les molécules de guidage ou les anticorps.

9. Procédé selon la revendication 1 ou 2, dans lequel le milieu de congélation comprend le diméthylsulfoxyde (DMSO), le tréhalose, la glycérine ou l'hydroxyéthylamidon (HES).

10. Procédé selon la revendication 1 ou 2, dans lequel les cellules adhérentes sont des cellules eucaryotes ou procaryotes ou des cellules eucaryotes ou procaryotes génétiquement modifiées.

11. Procédé selon la revendication 10, dans lequel les cellules eucaryotes sont des cellules végétales, animales ou fongiques.

12. Procédé selon la revendication 11, dans lequel les cellules animales sont des cellules de mammifère, soit des cellules primaires, soit des cellules immortalisées, de préférence des cellules humaines.

13. Procédé selon l'une quelconque des revendications 10 à 12, dans lequel les cellules sont choisies parmi les cellules souches telles que les lignées de cellules souches embryonnaires non humaines, les cellules souches pluripotentes induites, les cellules souches mésenchymateuses, les cellules souches endothéliales, les cellules souches hématopoïétiques, les cellules souches neurales, les cellules souches de la crête neurale ou les cellules souches gastro-intestinales ; les cellules somatiques telles que les cellules endothéliales, les cellules épithéliales de l'estomac et de l'intestin, les cellules épithéliales de mammifères, les mélanocytes, les cellules épithéliales pulmonaires, les cellules épithéliales rénales, les kératinocytes, les cellules urothéliales, les hépatocytes, les cellules de la lentille cornéenne, les ostéoblastes, les ostéocytes, les odontoblastes, les chondrocytes, les cellules ligamentaires, les cellules tendineuses, les cellules de l'hypophyse, les cellules des glandes salivaires, les cellules des glandes surrénales, les cellules pancréatiques exocrines et endocrines, les cellules de Leydig, les cellules graisseuses, les fibroblastes, les cellules de la rétine, les neurones dopaminergiques, les neurones GABAergiques, les neurones glutaminergiques, les neurones cholinergiques, les neurones adrénergiques, les astrocytes, les oligodendrocytes, les cellules de Schwann, les cellules des muscles lisses, les cellules des muscles squelettiques, les cardiomyocytes, les lymphocytes B, les lymphocytes T, les macrophages, les neutrophiles, les cellules dendritiques, les mastocytes, les éosinophiles, les basophiles, les cellules tueuses naturelles, les cellules M ou la microglie ; les cellules génétiquement modifiées ; les lignées de cellules cancéreuses telles que les cellules HeLa, CCF-STTG1, Hep G2, SK-MEL-5, Saos-2 ou WERI-Rb-1 ; les lignées de cellules immortalisées telles que NuLi, CuFi, CHON-001, BJ-5ta, hTERT-HME1 (ME16C), hTERT-HPNE, hTERT RPE-1, NeHepLxHT ou HESC ; les cellules végétales telles que les cellules de Brownanthus corallinus, Carpanthea pomeridiana, Conophytum meyerae, Delosperma ecklonis, Sesuvium portulacastrum, Sphalmanthus trichomotus, Amaranthus retroflexus, Pleuropetalum darwinii, Amaryllidaceae, Crinum x powellii hort., Catharanthus longifolius, Dictyophleba leonensis ou Ervatamia coronaria ; les cellules fongiques telles que Dacrymyces deliquescens, Fennellomyces linderi, Cunninghamella blakesleeana, Tolypocladium inflatum, Radiomyces spectabilis, Wallemia sebi, Lophodermium seditiosum, Saccharomyces cerevisiae, Candida albicans, Debaromyces japonicus, Fellomyces polyborus, Brettanomyces abstinens, Hyphopichia burtonii ou Kloeckera brevis ; les cellules bactériennes telles que Acidobacterium capsulatum, Escherichia coli, Saccharococcus thermophilus, Lactobacillus acidophilus, Bacillus thuringiensis, Pseudomonas aeruginosa ou Enterococcus faecalis.

14. Assemblage de cellules-support cellulaire de collagène congelé ou construction de cellules artificielle congelée ou assemblage de tissus complexe tridimensionnel congelé pouvant être obtenu(e) par le procédé selon la revendication 1 ou 2, dans lequel/laquelle l'assemblage de cellules-support cellulaire de collagène congelé ou la construction de cellules artificielle congelée ou l'assemblage de tissus complexe tridimensionnel congelé présente la même structure tridimensionnelle après décongélation.

15. Assemblage de cellules-support cellulaire de collagène ou construction de cellules artificielle ou assemblage de tissus complexe tridimensionnel décongelé(e) selon la revendication 14, pour une utilisation dans le domaine de la médecine régénérative.

16. Utilisation d'un assemblage de cellules-support cellulaire de collagène décongelé, d'une construction de cellules artificielle décongelée ou d'un assemblage de tissus complexe tridimensionnel décongelé selon la revendication 14, pour la recherche de base ou pour des systèmes de test in vitro tels que des systèmes de test pour des substances pharmacologiques, des produits chimiques ou des produits cosmétiques.
